# EUROPÄISCHE PATENTSCHRIFT

(11) **EP 1 832 309 B1**
(45) Veröffentlichungstag und Bekanntmachung des Hinweises auf die Patenterteilung: **03.06.2009**
(21) Anmeldenummer: 07003085.3
(22) Anmeldetag: 14.02.2007
(51) Int. Cl.: A61N 1/05

(54) **Implantierbare medizinische Elektrodenvorrichtung, insbesondere kardiovaskuläre Herzschrittmacher- oder Defibrillator-Elektrodenvorrichtung**
Implantable medical electrode device, in particular cardiovascular pacemaker or defibrillator electrode device
Dispositif médical implantable à électrodes, en particulier dispositif de défibrillation ou de stimulation cardiovasculaire à électrodes

(30) Priorität: 11.03.2006 DE 102006011349
(43) Veröffentlichungstag der Anmeldung: 12.09.2007
(73) Patentinhaber: BIOTRONIK CRM Patent AG, 6341 Baar (CH)
(72) Erfinder: Dreier, Eckard, 10711 Berlin (DE); Flach, Erhard, 12305 Berlin (DE); Geistert, Wolfgang, Dr., 79618 Rheinfelden (DE); Maxfield, Michelle, 10967 Berlin (DE); Palm, Jochen, 15831 Mahlow (DE); Schurr, Marc, 13359 Berlin (DE); Brinkmann, Jan-Heiner, 12623 Berlin (DE)
(74) Vertreter: Lindner-Vogt, Karin L.

(56) Entgegenhaltungen:
- EP-A- 1 062 972
- EP-A1- 0 261 582
- EP-A1- 0 546 414
- EP-A1- 0 779 080
- WO-A-98/42403
- DE-C1- 3 718 139
- US-A- 3 937 225
- US-A- 4 913 164
- US-A1- 2006 036 307

## Beschreibung

Die Erfindung betrifft eine implantierbare medizinische Elektrodenvorrichtung und insbesondere eine kardiovaskuläre Herzschrittmacher- oder Defibrillator-Elektrodenvorrichtung.

Zum Hintergrund der Erfindung ist festzuhalten, dass derartige implantierbare Elektrodenvorrichtungen an einer bestimmten Körperstelle zu verankern sein müssen, damit sie ihre Position im Laufe der Zeit nicht verändern. Besonders wichtig ist dies, wenn solche Elektrodenvorrichtungen in einem sich bewegenden Organ, wie z. B. im Herzen, implantiert werden.

Für die Festlegung der Elektrodenvorrichtung ist es bekannt, an deren langgstreckten, schlauchartigen Elektrodenkörper in einer Fixierzone vor dem distalen Ende Fixiereinrichtungen, wie Schrauben, Nadeln, Haken, Anker (so genannte Tines) oder Vorsprünge und entsprechende Hinterschneidungen auszubilden, in die das Gewebe einwachsen kann.

Eine Besonderheit stellt die Fixierung in einem Blutgefäß dar, da insbesondere hier scharfe Spitzen oder Kanten der Fixiereinrichtung Verletzungen oder zumindest Reizungen an der Gefäßwand hervorrufen können. Aus diesem Grund sind herkömmliche Fixiereinrichtungen so ausgelegt, dass sie sich durch ihre Formgebung im Gefäß verklemmen.

Neben der möglichst atraumatischen Verankerung solcher Elektrodenvorrichtungen spielt auch die Fixierung in Gefäßen verschiedener Durchmesser eine Rolle.

Marktgängig sind in Gefäßen implantierbare Elektrodenvorrichtungen, welche zur Fixierung die oben erwähnten "Tines", Silikonschrauben oder helix- bzw. hakenförmige Elektrodenkörper aufweisen. Durch derartig vorgeformte Elektrodenkörper, beispielsweise mit gezielt angebrachten Knickstellen, kann sich der Elektrodenkörper formschlüssig an die Winkel und Abgänge von beispielsweise Venen im Herzgefäßsystem anlegen und damit eine Fixierung erreichen. In geraden Venen kann durch die Streckung dieser gezielten Knicke eine Lagefixierung erfolgen. Ferner sind Elektroden in Anwendung, die distal einen als Helix geformten Elektrodenkörper besitzen, der sich an der Gefäßwand durch Expansionskräfte festspannt.

Eine besonders einfache, therapeutisch dabei jedoch nicht die optimalen Ergebnisse erzielende Fixierungstechnik ist ein Verkeilen der distalen Spitze des Elektrodenkörpers in einem Gefäß. Dabei wird der Elektrodenkörper so weit beispielsweise in den linksventrikulären Venenästen vorangeschoben, bis die Spitze des Elektrodenkörpers in der Vene feststeckt und eine "Verkeilungsposition" ("wedge position") einnimmt. In dieser wird die Vene verschlossen.

Alle vorstehenden Verankerungstechniken haben verschiedene Nachteile, wie beispielsweise die Abhängigkeit der Endposition des Elektrodenkörpers vom Verhältnis der Durchmesser der Vene einerseits zu den Dimensionen der Spitzen, Helix-Verankerungen oder "Tines" andererseits. Die damit erreichbare Endposition des Elektrodenkörpers muss nicht unbedingt die therapeutisch günstigste Position sein.

Zum druckschriftlichen Stand der Technik ist beispielsweise auf die WO 98/42403 A1 oder die US 5,170,802 A zu verweisen. Diese Druckschriften offenbaren eine Fixiereinrichtung mit einem so genannten "Stent", der expandiert wird, wenn sich die Elektrodenvorrichtung an der gewünschten Position im Gefäß befindet. Durch die Expansion der Stentstruktur wird der Elektrodenkörper in der Position festgeklemmt. Nachteil dieser Lösung ist, dass die Fixierung nicht reversibel ist, was ein manchmal notwendiges Lösen und Umplatzieren des Elektrodenkörpers nach einer ersten Fixierung und auch eine Entfernung des Elektrodenkörpers schwierig machen.

In der WO 94/07564 A1 wird eine Lösung für das Problem der Umplatzierbarkeit offenbart, bei der die Fixierung durch einen expandierbaren oder selbst expandierenden Drahtkorb erfolgt. Nachteil dieser Konstruktion ist, dass die Drähte in die Gefäßwand einschneiden und somit sehr traumatisch sein können.

Die US 5,411,546 A1 schließlich zeigt verschiedene Lösungen für die Fixierung in einem Gefäß, so beispielsweise reversibel aus der Katheterhülle ausfahrbare und wieder darin rückziehbare Drahtspiralen. Durch den kleinen Durchmesser der Drähte und die damit verbundene geringe Kontaktfläche zwischen Spirale und Gefäßwand und die nur begrenzte Spannkraft, mit der die Spirale gegen die Gefäßwand drücken darf, damit letztere nicht geschädigt wird, ist die Fixierung allerdings nicht sehr zuverlässig. Die ferner in der Druckschrift offenbarten Drahtkonstruktionen sind - wie bereits oben erwähnt - hoch-traumatisch. Ferner ist in diesem druckschriftlichen Stand der Technik noch eine so genannte "Segelkonstruktion" gezeigt, die wiederum nicht reversibel ist.

Weiterhin wird auf die US 2006/0036307 A1 hingewiesen. Diese offenbaren implantierbare kardiovaskuläre Herzschrittmacher- oder Defibrillator-Elektrodenvorrichtung, die einen langgestreckten, schlauchartigen Elektrodenkörper und eine Fixierzone im distalen Endbereich des Elektrodenkörpers umfassen. Die Fixierzone hat eine nach außen geschlossenene Umfangshülle und dient der lösbaren Fixierung der Elektrodenvorrichtung in einem Körper-Lumen, indem sie reversibel expandierbar ist. Die Steuerung der Expansion oder Kontraktion wird durch ein in der Fixierzone angeordnetes Expansionsmittel gewährleistet.

Zusätzlich zu diesen Merkmalen zeigt die EP 0 546 414 A1 eine Elektrodenvorrichtung, bei der das Expansionsmittel durch pneumatische oder hydraulische Druckbeaufschlagung gebildet ist, mit deren Hilfe die in der Fixierzone flexible Wandung (8) des Elektrodenkörpers reversibel expandierbar ist.

Jedoch ist eine permanent dichte Wandung schwer herzustellen, so dass es sich gezeigt hat, dass die genannten Lösungen mit der Zeit ihre expandierte Form und damit die Fixierung verlieren. Weiterhin ist die Expansionskraft der flexiblen Wandung begrenzt.

Davon ausgehend liegt der Erfindung die Aufgabe zugrunde, eine Elektrodenvorrichtung zu schaffen, die einerseits eine zuverlässige, andererseits aber auch reversible Fixierung mit ausreichender Expansionskraft ermöglicht und dabei vergleichsweise atraumatisch ist.

Diese Aufgabe wird erfindungsgemäß durch die im Anspruch 1 angegebenen Merkmale der Elektrodenvorrichtung gelöst, wonach der Elektrodenkörper im Bereich seiner Fixierzone mit einer nach außen geschlossenen Umfangshülle versehen ist, die jedoch zur lösbaren Fixierung in einem Körper-Lumen reversibel expandierbar ist. Zur Steuerung der Expansion ist in der Fixierzone ein Expansionsmittel vorgesehen, das unterschiedlichst ausgebildet sein kann. Zu der nach außen geschlossenen Umfangshülle ist festzuhalten, dass es sich dabei um die Wandung des eigentlichen Elektrodenkörpers der Elektrodenvorrichtung, aber auch um eine gesonderten, über den Elektrodenkörper gezogenen Schlauch handeln kann. In der Wandung im Bereich der Fixierzone ist die Elektrodenleitung mit einer stentartigen, plastisch verformbaren Stützstruktur versehen, die durch Druckbeaufschlagung in einen stationär expandierten Fixierzustand und durch Unterdruckbeaufschlagung in einen kontrahtierten Ausgangszustand überführbar ist.

Aufgrund der vorstehenden erfindungsgemäßen Konstruktion der Fixierzone ist eine sichere, atraumatische Fixierung der Elektrodenvorrichtung in beispielsweise einem Venengefäß im Herzen möglich, indem die Umfangshülle durch das Expansionsmittel (wie beispielsweise Fluiddruck, einen Ballon oder axialer Zug/Druck auf die Fixierzone) an der gewünschten Position so weit expandiert wird, bis die Umfangshülle sich im Gefäß festlegt. Erkennbar erfolgt eine Fixierung also - innerhalb gewisser Grenzen natürlich - unabhängig vom Durchmesser des Körper-Lumens. Da eine nach außen geschlossene Hülle expandiert, stehen in der Fixierzone keine scharfen Drähte, Ecken oder Kanten ab, sodass die Fixierung - wie aufgabengemäß vorgesehen - sehr atraumatisch erfolgt.

In den Unteransprüchen sind bevorzugte Ausführungsformen der expandierbaren Fixierzone mit alternativen Expansionsmitteln angegeben. Zur Vermeidung von Wiederholungen wird zu näheren Ausführungen hierüber auf die nachfolgende Beschreibung verwiesen, in der verschiedene Ausführungsbeispiele des Erfindungsgegenstandes anhand der beigefügten Zeichnungen näher erläutert werden. Es zeigen:
- Fig. 1 und 2: einen Längsschnitt durch die Fixierzone einer Elektrodenvorrichtung in kontrahiertem bzw. expandiertem Zustand der Fixierzone,
- Fig. 3: eine schematische Gesamtansicht einer Elektrodenvorrichtung,
- Fig. 4: einen Detail-Axialschnitt der Elektrodenvorrichtung im Bereich der proximalen Druckmedium-Zuführung,
- Fig. 5: eine Teil-Seitenansicht der Fixierzone eines Elektrodenkörpers mit Verankerungselementen an der Außenseite,

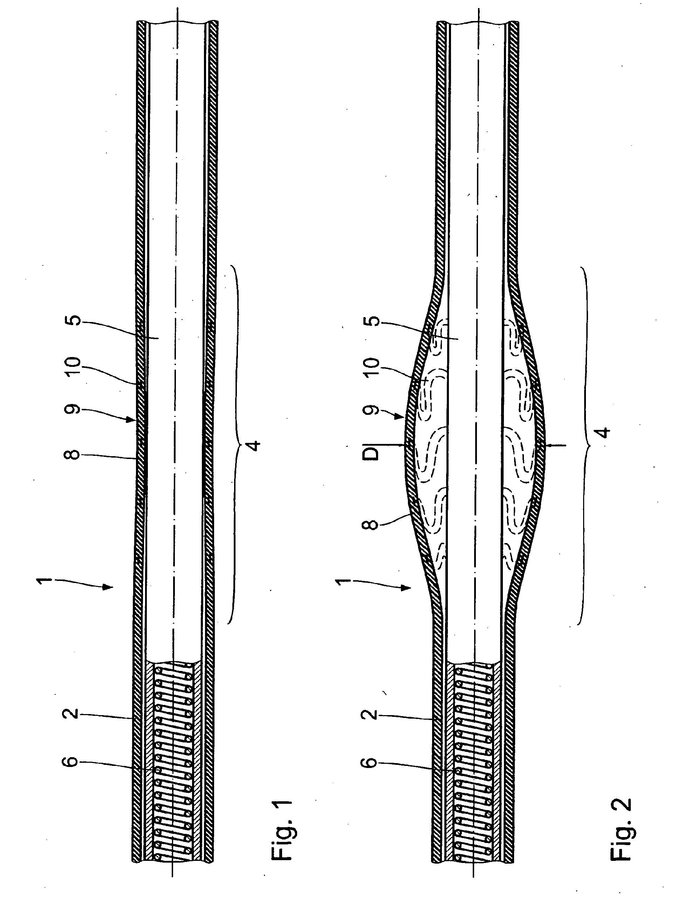

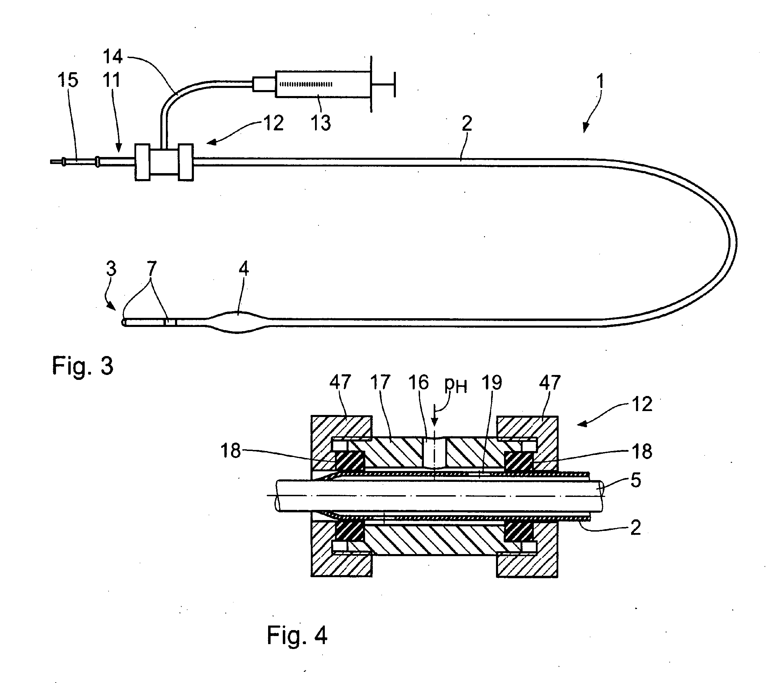

Wie aus Fig. 1 und 2 deutlich wird, weist eine implantierbare medizinische Elektrodenvorrichtung in Form eines als Ganzem mit 1 bezeichneten Herzkatheters einen langgestreckten, schlauchartigen Elektrodenkörper 2 auf, der vor dem distalen Ende 3 (Fig. 3) mit einer einige Millimeter langen Fixierzone 4 versehen ist. Im Inneren des Elektrodenkörpers 2 laufen in einem gesonderten Innenschlauch 5 die gewendelten Elektrodenzuleitungen 6, mit der die beispielsweise in Fig. 3 gezeigten Stimulationselektroden 7 mit Spannung versorgt werden.

Im Bereich der Fixierzone 4 ist die Wandung 8 des Elektrodenkörpers 2 flexibler als in den angrenzenden restlichen Bereichen ausgebildet. Ferner ist darin eine stentartige, plastisch verformbare Stützstruktur 9 in das Wandungsmaterial eingebettet. Diese Stützstruktur 9 ist ein im Wesentlichen rohrförmiges, plastisch verformbares, expandierbares Streckmetall- oder Kunststoffformteil. Die Expansionskräfte dieser Stützstruktur 9 sind durch geeignete Materialwahl und Strukturierung der Stege 10 - etwa durch Abstimmung der Stegbreite, Steghöhe, der Form der Biegeschenkel usw. - auf die elastischen und geometrischen Eigenschaften des einbettenden Materials der Wandung 8 der Fixierzone 4 abzustimmen, die wiederum durch die Wandstärke und die Materialwahl, wie beispielsweise Latex, Silikon, Polyurethan, bestimmt ist.

Wie aus Fig. 3 deutlich wird, ist am proximalen Ende 11 des Herzkatheters 1 eine Zuführeinrichtung 12 um den Elektrodenkörper 2 herum vorgesehen, über die mittels einer Spritze 13 über die angeschlossene Einspeiseleitung 14 ein hydraulisches Druckmedium, wie beispielsweise physiologische Kochsalzlösung, in den als druckdichter Schlauch ausgebildeten Elektrodenkörper 2 mit einem Druck p_{H} einpressbar ist. Das proximale Ende 11 des Herzkatheters 1 ist durch einen Anschlussstecker 15 für die einzelnen Stimulationselektroden 7 gebildet.

Die Zuführeinrichtung 12 ist in Fig. 4 dargestellt. Der Anschluss 16 der Einspeiseleitung 14 sitzt mittig in der Wand einer den Elektrodenkörper 2 umgebenden Zylinderhülse 17, zwischen deren Stirnöffnungen und dem Elektrodenkörper 2 jeweils O-Ring-Dichtungen 18 eingepasst sind. Diese werden von auf die Zylinderhülse 17 aufschraubbaren Klemmhülsen 47 zur Abdichtung beaufschlagt, sodass über den Anschluss 16 und eine Einspeiseöffnung 19 das Druckfluid in den Ringraum zwischen dem Elektrodenkörper 2 und dem Innenschlauch 5 eingepresst werden kann.

Wie aus Fig. 1 und 2 deutlich wird, wird der Herzkatheter 1 beispielsweise in den Koronarsinus mit seinem distalen Ende 3 vorgeschoben, bis die gewünschte Position der Stimulationselektroden 7 erreicht ist. Die Röntgenüberwachbarkeit dieses Vorgangs ist durch eine entsprechende Auslegung der Stützstruktur 9 aus einem röntgensichtbaren Material, wie beispielsweise geeignet beschichtetem Kunststoff, Edelstahl, Platin-, Titan-Legierungen, Magnesium oder Gold verbesserbar.

Durch Beaufschlagen der Fixierzone 4 mit dem Druckfluid als Expansionsmittel wird dort die Stützstruktur 9 plastisch aufgeweitet und dehnt sich damit radial aus, wie in Fig. 2 erkennbar ist. Dies wird so lange fortgesetzt, bis die Fixierzone 4 mit ihrem erweiterten Durchmesser an der Gefäßinnenwand (hier nicht dargestellt) anliegt und damit den Herzkatheter 1 in dieser Position fixiert.

Zu einer Umpositionierung oder Entfernung des Herzkatheters 1 wird das Druckfluid abgesaugt, wodurch ein Unterdruck entsteht, der dafür sorgt, dass die Fixierzone 4 sich wieder zusammenzieht und dabei die Stützstruktur 9 quasi kollabiert, womit wieder die in Fig. 1 gezeigte Konfiguration erreicht wird und der Herzkatheter 1 frei verschiebbar ist.

Der Expansionsdurchmesser D in der Fixierzone 4 kann durch Kontrolle des eingebrachten Volumens des Druckfluids oder durch den angelegten Druck - auch bei einer pneumatischen Steuerung - direkt beeinflusst werden. Das Druckfluid kann dabei auch durch ein exzentrisches Zusatzlumen im Elektrodenkörper 2 oder durch einen äußeren, koaxial angeordneten Schlauch übertragen werden, wie in den Figuren im Einzelnen nicht dargestellt ist.

Schließlich ist darauf hinzuweisen, dass durch eine geeignete Formgebung der eingebetteten Stützstruktur 9 auch eine axiale Verformbarkeit erreichbar ist, sodass der Herzkatheter 1 zur Platzierung in den Gefäßen auch im Bereich der Fixierzone 4 ausreichend flexibel bleibt.

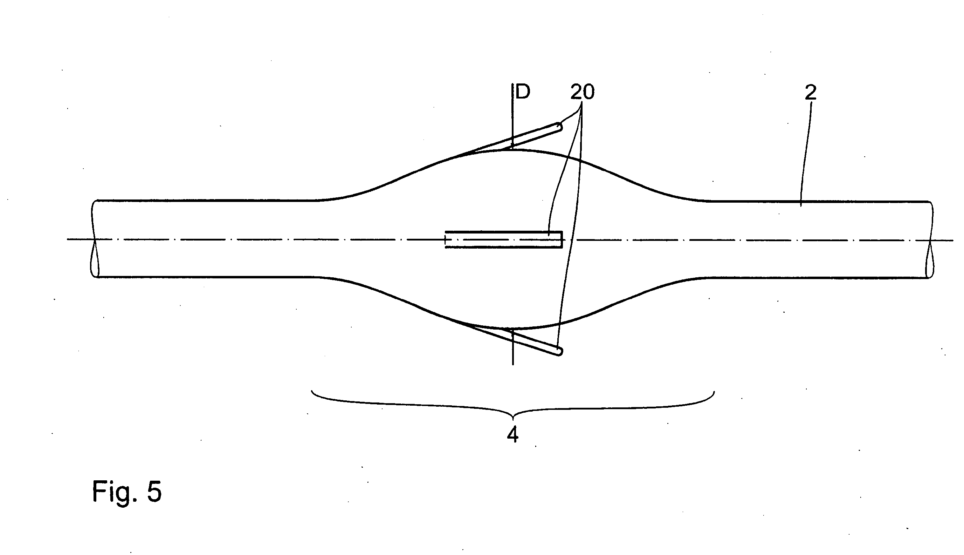

Wie aus Fig. 5 deutlich wird, kann durch eine geeignete Strukturierung der Außenfläche der Fixierzone 4 eine zusätzliche Festlegung des Herzkatheters 1 erreicht werden. Dazu sind in einer weiteren Ausführungsform im Bereich der Fixierzone 4 an der Außenseite stegförmige Verankerungselemente 20 über den Umfang verteilt parallel zur Längsrichtung des Elektrodenkörpers 2 angebracht. In der nicht expandierten Konfiguration liegen diese Verankerungselemente 20 flach an der Außenseite des Herzkatheters 1 an, sodass eine problemlose Einführung gewährleistet ist. Durch das Expandieren der Fixierzone 4 spreizen sich die Verankerungselemente 20 ab, wie es in Fig. 5 gezeigt ist und beaufschlagen zusätzlich mechanisch die Innenwand des hier nicht gezeigten Gefäßes. Damit wird insbesondere eine deutlich größere Auszugssicherheit des Herzkatheters 1 erreicht. Die Verankerungselemente 20 können je nach Materialauswahl weich oder steif ausgelegt sein. Als Materialien kommen Elastomere, andere Kunststoffe oder Metalle in Frage.

## Patentansprüche

1. Implantierbare medizinische Elektrodenvorrichtung, insbesondere kardiovaskuläre Herzschrittmacher- oder Defibrillator-Elektrodenvorrichtung umfassend
- einen langgestreckten, schlauchartigen Elektrodenkörper (2),
- eine Fixierzone (4) vor dem distalen Ende (3) des Elektrodenkörpers (2), in der der Elektrodenkörper (2) mit seiner nach außen geschlossenen Umfangshülle (8) zur lösbaren Fixierung der Elektrodenvorrichtung (1) in einem Körper-Lumen (27) reversibel expandierbar ist, und
- ein in der Fixierzone (4) angeordnetes Expansionsmittel zur Steuerung der Expansion und Kontraktion in der Fixierzone (4), welches durch pneumatische oder hydraulische Druckbeaufschlagung gebildet ist, mit deren Hilfe die in der Fixierzone (4) flexible Wandung (8) des Elektrodenkörpers (2) reversibel expandierbar ist,
**dadurch gekennzeichnet, dass**
der Elektrodenkörper (2) in seiner Wandung (8) im Bereich der Fixierzone (4) mit einer stentartigen, plastisch verformbaren Stützstruktur (9) versehen ist, die durch Druckbeaufschlagung in einen stationär expandierten Fixierzustand und durch Unterdruckbeaufschlagung in einen kontrahtierten Ausgangszustand überführbar ist.

2. Elektrodenvorrichtung nach Anspruch 2, **dadurch gekennzeichnet, dass** zur Druckbeaufschlagung der Fixierzone (4) der Elektrodenkörper (2) druckdicht ausgeführt ist und ein von seinem proximalen Ende (11) her zuleitbares Druckmedium führt.

3. Elektrodenvorrichtung nach einem der vorgenannten Ansprüche, **dadurch gekennzeichnet, dass** in der Fixierzone (4) außen am Elektrodenkörper (2) Verankerungselemente (20) angebracht sind, die in der expandierten Stellung der Fixierzone (4) mit der Wand des Körper-Lumens (27) in Eingriff stehen.

## Claims

1. An implantable medical electrode device, in particular a cardiovascular pacemaker or defibrillator electrode device, comprising
- an elongated tube-shaped electrode body (2),
- a fixation zone (4) upstream from the distal end (3) of the electrode body (2), in which the electrode body (2) is reversibly expandable for detachable fixation of the electrode device (1) with its peripheral sleeve (8) that is closed toward the outside in a lumen (27) of the body, and
- expansion means arranged in the fixation zone (4) for controlling the expansion and contraction in the fixation zone (4), which is formed by pneumatic or hydraulic pressure actuation with the help of which the flexible wall (8) of the electrode body (2) is reversibly expandable in the fixation zone (4),
**characterized in that**
the electrode body (2) is provided with a stent type plastically deformable supporting structure (9) in the area of the fixation zone (4) in its wall (8), said supporting structure being convertible to a stationary expanded fixation state by the action of pressure and being convertible into a contracted starting state by the action of a vacuum.

2. The electrode device according to Claim 2,
**characterized in that** the electrode body (2) is designed to be pressure-tight for the action of the pressure in the fixation zone (4) and to carry a hydraulic medium that can be supplied from its proximal end (11).

3. The electrode device according to any one of the preceding claims,
**characterized in that** anchoring elements (20), which are engaged with the wall of the lumen (27) of the body in the expanded position of the fixation zone (4), are attached to the outside of the electrode body (2) in the fixation zone (4).

## Revendications

1. Dispositif d'électrodes médical implantable, en particulier dispositif d'électrodes pour pacemaker cardiovasculaire ou dispositif d'électrodes pour défibrillateur cardiovasculaire comprenant
- un corps d'électrode (2) étiré en longueur et de type flexible,
- une zone de fixation (4) avant l'extrémité (3) distale du corps d'électrode (2), dans laquelle le corps d'électrode (2) peut être détendue de façon réversible avec son enveloppe périphérique (8) fermée vers l'extérieur pour la fixation amovible du dispositif d'électrodes (1) dans un lumen de corps (27), et
- un moyen de détente disposé dans la zone de fixation (4) pour la commande de la détente et de la contraction dans la zone de fixation (4), qui est formé par une alimentation en pression pneumatique ou hydraulique, à l'aide de laquelle la paroi (8), flexible dans la zone de fixation (4), du corps d'électrode (2) peut être détendue de façon réversible,
**caractérisé en ce que**
le corps d'électrode (2) est doté dans sa paroi (8) dans le secteur de la zone de fixation (4) d'une structure de soutien (9) de type stent, plastiquement déformable, qui peut être transféré par une alimentation en pression dans un état détendu de façon fixe et par une alimentation de dépression dans un état initial contracté.

2. Dispositif d'électrodes selon la revendication 2, **caractérisé en ce que** le corps d'électrode (2) est réalisé de façon étanche à la pression pour l'alimentation en pression de la zone de fixation (4) et guide un agent de pression pouvant être acheminé à partir de son extrémité (11) proximale.

3. Dispositif d'électrodes selon l'une quelconque des revendications précédentes, **caractérisé en ce que** dans la zone de fixation (4) sont placés à l'extérieur sur le corps d'électrode (2) des éléments d'ancrage (20) qui sont en prise, dans la position détendue de la zone de fixation (4), avec la paroi du lumen du corps (27).
